Europäisches Patentamt

European Patent Office

Office européen des brevets

(11) Publication number: 0 319 690
A1

## EUROPEAN PATENT APPLICATION

(21) Application number: 88117717.4

(22) Date of filing: 25.10.88

(51) Int. Cl.4: **C12N 15/00** , //(**C12N15/00,**
**C12R1:225)**

(30) Priority: 24.11.87 JP 294199/87
14.10.88 JP 25767/88

(43) Date of publication of application:
14.06.89 Bulletin 89/24

(84) Designated Contracting States:
CH DE FR GB IT LI NL SE

(71) Applicant: NISSHIN FLOUR MILLING CO., LTD.
19-12, Nihonbashi-koami-cho
Chuo-ku, Tokyo 103(JP)

Applicant: RIKAGAKU KENKYUSHO
2-1 Hirosawa
Wako-shi Saitama-ken(JP)

(72) Inventor: Ohmori, Takemitsu
4-8, Suehiro-cho 3-chome
Kawagoe-shi Saitama-ken(JP)
Inventor: Natori, Yohei
7-23, Fujimidai 3-chome
Nerima-ku Tokyo(JP)
Inventor: Imamoto, Fumio
4-524, Hibarigaoka 3-chome
Kashiwa-shi Chiba-ken(JP)
Inventor: Kano, Yasunobu
4-557, Hibarigaoka 3-chome
Kashiwa-shi Chiba-ken(JP)

(74) Representative: Türk, Gille, Hrabal
Bruckner Strasse 20
D-4000 Düsseldorf 13(DE)

(54) Method of introducing foreign DNA into certain bacteria.

(57) Disclosed is a method of introducing foreign DNA into the cells of bacteria belonging to the genus Lactobacillus or Bifidobacterium by way of electroporation. The method comprises applying pulse currents to a suspension containing the cells of bacteria belonging to said genus and a foreign DNA.

EP 0 319 690 A1

## METHOD OF INTRODUCING FOREIGN DNA INTO CERTAIN BACTERIA

### FIELD OF THE INVENTION

The present invention relates to a method of introducing foreign DNA into the cells of certain bacteria, namely those belonging to the genus Lactobacillus or Bifidobacterium. More particularly, it relates to a method of introducing foreign DNA into the cells of bacteria belonging to the genus Lactobacillus or Bifidobacterium by way of electroporation.

### BACKGROUND OF THE INVENTION

Hithertofore, various attempts have been made of introducing foreign DNA into host microorganisms in order to achieve expression by the host microorganism of the genetic information contained in the foreign DNA or genes. As the results of those attempts, there have been presented various methods for obtaining transformants into which foreign genes have been introduced. The introduction of foreign genes, however, is influenced by various factors which are intrinsic to the microorganism concerned, and therefore, a method which is applicable to certain microorganism may not necessarily be applied to other microorganism. For example, a method using calcium chloride is known for the introduction of foreign genes into Escherichia coli. Further, the protoplast method is known for the introduction of foreign genes into Bacillus subtilis or yeasts. However, the introduction of foreign genes into Lactobacillus or Bifidobacterium is difficult by the known methods described above. Therefore, it has been desired to obtain a method of introducing foreign genes into those bacteria.

Bacteria belonging to the genus Lactobacillus are important microbes that are widely used in various fields of industry, such as production of yoghurt, lactic acid drinks, dairy products (such as cheese), feedstuff and lactic acid. Thus, industry should be benefited if the bacteria of this genus can be altered artificially so that they may produce desired properties. The genetic manipulation of the bacteria of this genus is getting more and more important from the viewpoint of breeding.

As mentioned above, however, it has not been established so far to introduce foreign genes into bacteria of the genus Lactobacillus. The only methods known in this art are the conjugation method [A. W. Shrago et al., Appl. Environ. Microbiol., 52, pp574-576 (1986)] and the method of introducing foreign DNA to previously prepared protoplast [Japanese Patent LOP Publication No. 60-30686 as laid open to public inspection]. The conjugation method requires two different microbes, one being the DNA donor and the other being the DNA acceptor. The conjugation between the two microbes not only is complicated and accidental but also may not achieve with certainty the selective introduction of the desired genes into the DNA acceptor.

At the present stage, the conjugation method necessitates repeated experiments on a trial-and-error basis, and its result lacks reliability and reproducibility.

The protoplast method requires a prior step of producing protoplasts of Lactobacillus. The introduction rate of the desired DNA will be low if the production rate of protoplasts is low. Conversely, the viability of protoplast will be poor if the production rate of protoplasts is too high. Moreover, the protoplast method itself is not an established method for incorporating DNA in that it involves unsolved steps of producing protoplasts and restoring them.

Bacteria belonging to the genus Bifidobacterium are the microbes known to be a major member of the intestinal flora and known to affect the health conditions of the host animal. The inbalance of the bacteria in the intestines will bring about various symptoms such as diarrhea and dyspepsia, while the restoration of the balance will get rid of such symptoms. Attempts have been made to develop artificial methods for manipulation of this microbe in order to express the desired properties, only resulting in fail.

In the meantime, the electroporation method has recently been proposed as a method for introducing foreign genes into mammalian cells. According to the method, a suspension of host cells and foreign DNA is prepared in a vessel equipped with electrode pair. Then the high voltage pulse current is applied to the suspension to change the property of the cell membrane, such as physical damage. The foreign DNA is then introduced into the host cells through the membrane. The method is to utilize the local and temporary destruction of cell membrane, thereby increasing permeability of substance therethrough. The method has been applied to introduction of foreign genes into large cells such as animal and plant cells.

As regards the application of the electroporation method to microorganisms, there has been reported attempts for protoplast of yeasts and for bacteria, Bacillus cereus ["Introduction of genes into mammalian cells by electroporation method", Hiroko Inaba et al, Protein, Nucleic Acid and Enzyme, 32, No. 1, pp10-21, (1987)]. However, there appears to be no report so far about the applicability of the method to bacteria of the genus Lactobacillus and Bifidobacterium.

As described above, while there is a long-felt want to introduce foreign DNA into the cells of bacteria of the genus Lactobacillus and Bifidobacterium, any methods for introducing foreign DNA into cells known in the art may not have been applicable to these bacteria. Therefore, there is a strong desire for attaining a novel method of introducing foreign DNA into these bacteria.


## SUMMARY OF THE INVENTION

It is, therefore, an object of the invention to establish a novel method of introducing foreign DNA into bacteria of the genus Lactobacillus and Bifidobacterium, overcoming the difficulties involved in the genetic manipulation in the art.

According to the present invention, there is provided a method of introducing a foreign DNA into the cells of bacteria belonging to the genus Lactobacillus or Bifidobacterium, which comprises applying pulse currents to a suspension containing the cells of bacteria belonging to the said genus and a foreign DNA. In another embodiment, the suspension may further contain polyethylene glycol.


## DESCRIPTION OF THE INVENTION

There is no specific limitation as to the type of bacteria of the genus Lactobacillus to be transformed by the introduction of foreign DNA; any species belonging to the genus may be included. Representatives of individual species include, for example, Lactobacillus casei, Lactobacillus acidophilus, Lactobacillus bulgaricus, Lactobacillus lactis, Lactobacillus helveticus, Lactobacillus salivarius, Lactobacillus plantarum, Lactobacillus fermentum, Lactobacillus cellobiosus, Lactobacillus delbrueckii, Lactobacillus buchneri and Lactobacillus brevis.

Similarly, there is no specific limitation as to the type of bacteria of the genus Bifidobacterium to be transformed by the incorporation of foreign DNA; any species belonging to the genus may be included. Representatives of individual species include, for example, Bifidobacterium bifidum, Bifidobacterium infantis, Bifidobacterium breve, Bifidobacterium adolescentis, Bifidobacterium dentium, Bifidobacterium longum, Bifidobacterium pseudolongum, Bifidobacterium thermophilum, Bifidobacterium asteroides and Bifidobacterium indicum.

The method of this invention may be applied at any of the lag phase, the logarithmic phase and the stationary phase of bacteria of the genus Lactobacillus or Bifidobacterium.

Preferably, the method of the invention may be applied to bacteria at an early logarithmic phase. The term "logarithmic phase" means the stage when the logarithmic correlation of the number of the cells or the turbidity against the cultivation period becomes linear, namely the stage when the growth of bacteria becomes most active after the initial lag phase or induction phase when almost no growth of bacteria is observed.

The efficacy of introducing foreign genes into the host cells will be lower when the method of the invention is carried out at other phases. The early logarithmic phase may be determined by cultivating bacteria of the genus Lactobacillus or Bifidobacterium in a suitable medium, followed by plotting the logarithm of the turbidity against the cultivation time. Thus, the desired phase may be determined when the turbidity reaches within a predetermined range. For example, in the case of cultivation of Lactobacillus casei strain IAM 1045 in Briggs medium, the desired phase corresponds with the turbidity $A_{600}$ of from about 0.05 to about 1.0, preferably from about 0.05 to about 0.30. In a further exemplary case of cultivation of Bifidobacterium longum strain ATCC 15707 in Briggs medium, the desired phase corresponds also with the turbidity $A_{600}$ of from about 0.05 to about 1.0, preferably from about 0.05 to 0.30.

The cells thus produced may be collected by any conventional means, for example, centrifugation. Care must be taken not to damage the cells or impair the viability of the microbe. The collected cells are then dispersed and suspended in a suitable medium such as water, preferably distilled water, or a buffer solution which is known in the art and which is maintained normally in a range of pH value between 5.5 and 9, for example sodium phosphate buffer, trishydrochloric acid buffer or any of those in which sucrose is added.

The number of the cells in the suspension should preferably be high so as to fall within a range that allows the desired transformation. Then, a foreign DNA is added to the suspension of the cells. Alternatively, a suspension of the cells may be added to a foreign DNA. Generally, the amounts of the cells and the DNA in the suspension should be high enough to ensure the probability of the introduction of the DNA into the cells.

Normally, the mixed amount of 0.01 μg or higher per cuvette suffices.

The DNA, which is to be introduced into the host cells to achieve transformation, may be chosen from a wide range; for example, from relatively of small molecular weight of 2-3 Kb to of large molecular weight of 50-100 Kb or even higher. Such DNA includes, for example, pAMβ1 (Nos. 1 and 2); pBL1003 and pBL1027 (plasmids disclosed in Japanese Patent LOP Publication No. 62-155091 as laid open to public inspection) and pC194.

The suspension mixed with the foreign DNA is then subjected to electroporation. Generally, this may be performed by placing the suspension in a vessel equipped with electrode pair, then by applying the high voltage pulse to the suspension at a temperature of from about 0°C to about 40°C.

Various apparatuses for electroporation are commercially available, for example, under the trade names of Gene Pulser (Bio Rad Co., Ltd.), GTE-1 (Shimadzu Corp.), Beacon 2000 (Beacon Co., Ltd.), Progenator (Hoffer Science Instrument Co., Ltd.) and Zapper (Pds Inc.).

According to a preferred aspect of the invention, the method of this invention may be performed in the presence of a polyethylene glycol, whereby the transformation frequency may be enhanced. The polyethylene glycol includes, for example, polyethylene glycol 2000, 4000, 6000 and 20000. A higher concentration of the polyethylene glycol will result in better transformation frequency. Generally, the concentration of polyethylene glycol in the suspension is up to about 30% by weight to achieve a favorable efficiency; however, much higher concentration may be allowed. Although the suspension may contain inorganic substances such as magnesium chloride, calcium chloride or manganese chloride to a certain extent, a smaller content thereof will give better results.

The cells of the bacteria of the genus Lactobacillus or Bifidobacterium thus subjected to electroporation may be collected by conventional means. Namely, the transformant may be selected by utilizing such properties as resistance to antibiotics, assimilability of special sugars, requirements for amino acid, etc. Such properties have been previously contained in the DNA to be introduced into the host cells.

The present invention will be illustrated by the following Examples, which in no way are intended to limit the scope of the invention. In the Examples, extraction and identification of the plasmid from the erythromycin-resistant colony (hereinafter referred to as Em[R] colony) were carried out by the following steps.

Extraction of the plasmid

A loopful of Em[R] colony was inoculated into 5 ml of Briggs medium containing 1 μg/ml of erythromycin and the stationary culture was effected at 37°C overnight to give a seed culture. One ml of the seed culture was transplanted into 100 ml of Briggs medium (Example 1) containing 3 μg/ml of erythromycin and the stationary culture was effected at 37°C overnight. 100 ml of the cultured solution were transplanted into 900 ml of Briggs medium containing 3 μg/ml of erythromycin and the stationary culture was effected at 37°C for 4 hours. At the end of this time, the cells were collected by centrifugation at 6000 rpm for 5 minutes at a temperature of 4°C. The cells were suspended in TES (30 mM Tris-Cl + 50 mM NaCl + 5 mM EDTA, pH 8.0) and the cells were again collected by centrifugation. The cells were then suspended in 8 ml of an aqueous sucrose solution (25% sucrose / 50 mM Tris-Cl + 1 mM EDTA, pH 8.0), into which 2 ml of a lysozyme solution (10 mg of lysozyme in 1 ml of 0.25 M Tris-Cl, pH 8.0) were added, and then the mixture was kept at 37°C for 1 hour.

The mixture was cooled to 0°C, 3.2 ml of 0.25 M EDTA (pH 8.0) and subsequently 3.2 ml of 10% SDS (sodium laurylsulfate) were added, and the whole mixture was allowed to stand at the same temperature for 10 minutes. 5.6 ml of 5 M sodium chloride were added to the mixture and the whole mixture was slowly stirred and then allowed to stand at 0°C for 1 hour.

The mixture was centrifuged at 24000 rpm for 30 minutes at 4°C to give a supernatant, which was then mixed with an equal volume of a mixed phenol-ClAA solution [prepared from one volume of phenol saturated with TE (10 mM Tris-Cl + 1 mM EDTA, pH 8.0) and one volume of a 24 : 1 mixture of chloroform and isoamyl alcohol]. The mixture was shaken and then centrifuged at 9000 rpm for 10 minutes to give a supernatant. This procedure was repeated twice. An equal volume of CIAA was added to the supernatant thus obtained. The mixture was shaken and then centrifuged at 9000 rpm for 10 minutes to give a supernatant. 1/10 volume of 3 M aqueous solution of sodium acetate and 2.2 volumes of ethanol were added to the supernatant. The mixture was allowed to stand at -80°C for 1 hour and then centrifuged at 9000 rpm for 10 minutes. The precipitate was washed with 70% ethanol, dried and subjected to ultracentrifugation with cesium chloride at 99000 rpm for 16 hours. The band containing the plasmid was collected. After the salts were removed by passing it through a column containing Sephadex G-25, the residue was extracted three times with phenol, washed with diethyl ether and precipitated by the addition of ethanol. The precipitate was washed with 70% ethanol and dried to give a plasmid sample. The sample was dissolved in distilled water and the solution was subjected to the following test.

Identification of plasmid

1. Size of the plasmid:

The plasmid extracted from the Em$^R$ colony which was obtained by the procedure mentioned above and the pAMβ1 No. 1 plasmid were subjected to agarose gel electrophoresis. Their mobility was observed and the same mobility was assigned to the same molecular size.

2. Cleavage pattern with the restriction enzyme:

The plasmid extracted from Em$^R$ colony which was obtained by the procedure mentioned above and the pAMβ1 No. 1 plasmid were subjected to cleavage with a restriction endonuclease (such as EcoRI, PvuII, HindIII, KpnI, etc.). The digested fragments were subjected to agarose gel electrophoresis to compare each other and the same number of the fragments and the same size of the fragments were assigned to the same plasmids.

Example 1

Briggs medium having the following composition was prepared in a conventional manner.

| Composition: | |
|---|---|
| Extract of tomato juice* | 400 ml |
| Glucose | 20 g |
| Starch | 0.5 g |
| Yeast extract (available from Difco) | 6 g |
| Polypeptone (available from Daigo Eiyo K.K.) | 15 g |
| Sodium glutamate | 2 g |
| CH$_3$COONa.3H$_2$O | 10 g |
| KH$_2$PO$_4$ | 4 g |
| NaCl | 5 g |
| Distilled water | add up to a whole volume of 1000 ml |
| pH = 6.8 | |

*Extract of tomato juice was prepared by adding an equal volume of distilled water to a commercially available tomato juice (by Del Monte Co., Ltd.), heating the resultant mixture at about 60°C for one hour and further at 100°C for 5 minutes, cooling and removing insoluble materials by filtration.

The medium was employed, when used, after adding of a solution of 200 mg of cysteine and 3.4 g of sodium ascorbate in 10 ml of distilled water.

To 10 ml of the medium was inoculated 0.2 ml of a seed culture solution of Lactobacillus casei IAM 1045 strain, which had been stationary-cultured on the medium at 37°C overnight, and stationary culture was effected at 37°C. The bacteria at the early logarithmic phase ($A_{600}$ = 0.25) were collected from 20 ml of cultured solution by centrifugation at 3000 rpm for 10 minutes, washed with Buffer Solution I [7 mM sodium phosphate buffer (pH 7.0), 1 mM $MgCl_2$ and 0.4 M sucrose] at room temperature and then 0.25 ml of the same Buffer Solution I was added to suspend the bacteria therein. 0.1 ml of the resultant suspension, 0.3 ml of a 40% aqueous solution of polyethylene glycol 6000 and 4 μg of plasmid pAMβ1 No. 1 having erythromycin-resistant gene were admixed and the resulting mixture was allowed to stand for 10 minutes. To the mixture was applied an electric current at room temperature under the condition of 5 kv/cm and 1 μF by means of Gene Pulser available from Bio Rad Co., Ltd. After the application of electric current, the mixture was allowed to stand for 10 minutes and then centrifuged at 15000 rpm for 2 minutes to collect the bacteria.

The collected bacteria were suspended in 1 ml of said medium and maintained at 37°C for 2 hours. Thereafter, 0.3 ml of this suspension was coated onto an erythromycin-containing Briggs agar medium and incubated at 37°C under anaerobic condition for two days to produce 161 erythromycin-resistant colonies.

From the resultant erythromycin-resistant colonies was extracted a plasmid and the plasmid size and cleavage pattern by restriction endonuclease thereof were compared with and identical with those of the said pAMβ1 No. 1. This shows that pAMβ1 No. 1 was introduced into L. casei IAM 1045 strain.

Also, the same procedure as in the above steps was repeated except that pAMβ1 No. 1 was not incorporated, whereby there were not produced any erythromycin-resistant colony.

## Example 2

Following the same procedure as in Example 1 except that the bacteria at the early logarithmic phase ($A_{600}$ = 0.2) and Buffer Solution II [7 mM Tris-hydrochloric acid buffer (pH 8.0), 1 mM $MgCl_2$ and 0.4 M sucrose] were substituted for the bacteria of Example 1 and the Buffer Solution II, respectively, there were produced 40 erythromycin-resistant colonies.

A plasmid was extracted from the resulting colonies in the same manner as stated above and was then confirmed to be pAMβ1 No. 1.

## Example 3

Following the same procedure as in Example 1 except that an electric condition by Gene Pulser was changed to that of 1.25 kv/cm and 24 μF, there were produced 68 erythromycin-resistant colonies.

A plasmid was extracted from these colonies and the plasmid was in agreement with pAMβ1 No. 1 with regard to the plasmid size and cleavage pattern by restriction endonuclease thereof. This shows that pAMβ1 No. 1 was introduced into L. casei IAM 1045 strain.

Also, the same procedure as in the above steps was repeated except that pAMβ1 No. 1 was not incorporated. There were not produced any erythromycin-resistant colony.

## Example 4

The same procedure as in Example 1 was repeated with various changes in collection periods of bacteria in Example 1, i.e. using the bacteria at different stages in growth phases of the bacteria. The results are shown below.

| | Number of the resultant erythromycin-resistant colonies | |
|---|---|---|
| Turbidity ($^A600$) | pAM$\beta$l No. 1 added | pAM$\beta$l No. 1 not added |
| 0.1 | 14 | 0 |
| 0.2 | 22 | 0 |
| 0.3 | 19 | 0 |
| 0.4 | 5 | 0 |

A plasmid was extracted from the resulting colonies in the same manner as stated above and was then confirmed to be pAM$\beta$1 No. 1.

### Example 5

The same procedure as in Example 1 was repeated except that the bacteria of $A_{600} = 0.2$ was used and the plasmid pAM$\beta$1 No. 2 wherein DNA is partially deleted from pAM$\beta$1 No. 1 was added in 1 $\mu$g based on the DNA amount in place of the collected bacteria and pAM$\beta$1 No. 1 of Example 1. There were produced 5 erythromycin-resistant colonies per one agar plate.

Plasmids were extracted from the resultant erythromycin-resistant colonies, respectively, and each was compared with pAM$\beta$1 No. 2, whereupon the plasmid size and cleavage pattern by restriction endonuclease thereof were found to be identical. This shows introduction of pAM$\beta$1 No. 2.

### Example 6

To 10 ml of the medium in Example 1 was inoculated 0.2 ml of a seed culture solution of L. casei IAM 1045 strain, which had been stationary-cultured overnight at 37°C in said medium, and stationary culture was effected at 37°C. From 80 ml of the culture solution were collected the bacteria of $A_{600} = 0.2$ by centrifugation at 3000 rpm for 10 minutes at 4°C. The bacteria were washed with 10 ml of a buffer solution at 0°C [8mM Tris-hydrochloric acid buffer (pH 8.5) and 0.4 M sucrose] and then 0.75 ml of the same buffer solution was added to suspend the bacteria therein. Thereafter, 0.1 ml of the resulting suspension, 2 $\mu$g of the plasmid pAM$\beta$1 No. 1 having erythromycin-resistant gene and 0.3 ml of a 40% aqueous solution of polyethylene glycol (PEG-6000) were admixed and the resulting mixture was allowed to stand at 0°C for 10 minutes. To the mixture was applied an electric current under the condition of 5 kv/cm and 1$\mu$F by means of Gene Pulser available from Bio Rad Co., Ltd. After the application of electric current, the mixture was allowed to stand at 0°C for 10 minutes and centrifuged at 15000 rpm for 2 minutes at 4°C to collect the bacteria.

A suspension of the collected bacteria in 1 ml of said medium was incubated at 37°C for 3 hours. Then, 0.3 ml of this suspension was coated onto an erythromycin-containing Briggs agar medium and incubation was effected at 37°C under anaerobic condition for two days to produce 1600 erythromycin-resistant colonies (a transformation frequency of $2.4 \times 10^{-5}$).

A plasmid was extracted from the colonies in the same manner as stated above and was confirmed to be pAM$\beta$1 No. 1.

Also, where the plasmid was not incorporated, there could not be produced any erythromycin-resistant colony.

### Example 7

The same procedure as in Example 6 was repeated except that a concentration of the polyethylene glycol (PEG 6000) was changed to 0, 5, 10, 15, 20 or 25% (the PEG% by weight in the suspension). The results are shown below.

| PEG (%) | Number of colonies/plate | Transformation frequency |
|---|---|---|
| 0 | 48 | $1.4 \times 10^{-6}$ |
| 5 | 96 | $2.0 \times 10^{-6}$ |
| 10 | 103 | $1.8 \times 10^{-6}$ |
| 15 | 130 | $1.9 \times 10^{-6}$ |
| 20 | 525 | $7.1 \times 10^{-6}$ |
| 25 | 1360 | $1.5 \times 10^{-5}$ |

A plasmid was extracted from the resulting colonies in the same manner as in Example 1 and confirmed to be pAM$\beta$1 No. 1. Also, where the plasmid was not incorporated, there could not be produced any resistant colony.

## Example 8

The same procedure as in Example 6 was repeated except that 0.4 $\mu$g of pAM$\beta$1 No. 1 was substituted for 2 $\mu$g of the same and the following sorts of polyethylene glycol (PEG) were used. The results are shown below.

| Sort of PEG | Number of colonies/plate | Transformation frequency |
|---|---|---|
| PEG 2000 | 480 | $6.2 \times 10^{-6}$ |
| PEG 4000 | 72 | $9.1 \times 10^{-7}$ |
| PEG 6000 | 68 | $8.6 \times 10^{-7}$ |
| PEG 20000 | 109 | $2.0 \times 10^{-6}$ |

A plasmid was extracted from the resultant colonies in the same manner as in Example 1 and confirmed to be pAM$\beta$1 No. 1. Also, where the plasmid was not incorporated, there could not be produced any resistant colony.

## Example 9

The same procedure as in Example 6 was repeated except that Lactobacillus acidophilus SS.27 strain (FERM 8551) (using PEG 6000 and Briggs medium containing 1 g/l of Tween 80) and L. casei JCM 1053 strain (using PEG 2000) were substituted for the L. casei IAM 1045 strain and the electric current was applied under the condition of 5 kv/cm and 1 $\mu$F.

With L. acidophilus SS.27 strain, there were produced 3 erythromycin-resistant colonies. With L. casei JCM 1053 strain, there were produced 27 erythromycin-resistant colonies.

Then, a plasmid was extracted from the resultant colonies in the same manner as in Example 1 and confirmed to be pAM$\beta$1 No. 1. Also, where the plasmid was not incorporated, there could not be produced any resistant colony.

## Example 10

The same procedures as in Example 6 were repeated except that L. acidophilus SS.27 strain and L. acidophilus ATCC 4356 strain (using Briggs medium containing 1 g/l of Tween 80) were substituted for the L. casei IAM 1045 strain, plasmid pC194 was substituted for the plasmid pAM$\beta$1 No. 1, a Briggs agar medium containing 5 $\mu$g/ml of chloramphenicol was substituted for the erythromycin-containing Briggs agar medium and the electric current was applied under the condition of 5 kv/cm and 1 $\mu$F.

With L. acidophilus SS.27 strain, there were produced 4 chloramphenicol-resistant colonies. With L. acidophilus ATCC 4356 strain, there were produced 12 chloramphenicol-resistant colonies.

Then, a plasmid was extracted from the resulting colonies in the same manner as in Example 1 and confirmed to be plasmid pC194.

Example 11

From a seed culture solution of L. casei IAM 1045 strain, which had been stationary-cultured at 37° C overnight in the same medium as in Example 6, were collected the bacteria by centrifugation at 3000 rpm for 10 minutes at 4° C. The collected bacteria were washed with 10 ml of a buffer solution [8 mM Tris-hydrochloric acid buffer (pH 8.5) and 0.4 M sucrose] at 0° C, and then 0.75 ml of the same suspension was added to suspend the bacteria therein. 0.1 ml of the resultant suspension, 2 μg of the plasmid pAMβ1 No. 1 having erythromycin-resistant gene and 0.3 ml of a 40 % aqueous solution of polyethylene glycol (PEG 6000) were admixed and the resulting mixture was allowed to stand at 0° C for 10 minutes. To the mixture was applied the electric current under the condition of 5 kv/cm and 1 μF by means of Gene Pulser available from Bio Rad Co., Ltd. After the application of electric current, the mixture was allowed to stand at 0° C for 10 minutes and centrifuged at 15000 rpm for 2 minutes, at 4° C to collect the bacteria.

A suspension of the collected bacteria in 1 ml of said medium was incubated at 37° C for 3 hours. Then, 0.3 ml of this suspension was coated onto an erythromycin-containing Briggs agar medium and incubation was effected anaerobically at 37° C for two days to produce 6 erythromycin-resistant colonies.

A plasmid was extracted from the colonies in the same manner as in Example 1 and confirmed to be pAMβ1 No. 1. Also, where the plasmid was not incorporated, there could not be produced any erythromycin-resistant colony.

Example 12

The same procedure as in Example 6 was repeated except that the bacteria-collecting period was variously changed, a concentration of polyethylene glycol was changed from 30% to 25% and a concentration of the plasmid was changed from 2 μg to 1.5 μg. The results are shown below.

| | pAMβl No. 1 added | | | pAMβl No. 1 not added |
|---|---|---|---|---|
| Turbidity ($^A$600) | Number of resulting erythromycin-resistant colonies | Transformation frequency | | Number of resulting erythromycin-resistant colonies |
| 0.1 | 440 | $5.3 \times 10^{-6}$ | | 0 |
| 0.2 | 880 | $1.3 \times 10^{-5}$ | | 0 |
| 0.3 | 1150 | $1.3 \times 10^{-5}$ | | 0 |
| 0.45 | 810 | $1.2 \times 10^{-5}$ | | 0 |
| 0.6 | 420 | $5.3 \times 10^{-6}$ | | 0 |
| 0.8 | 350 | $1.2 \times 10^{-6}$ | | 0 |
| 1.0 | 150 | $4.6 \times 10^{-7}$ | | 0 |

A plasmid was extracted from the colonies in the same manner as in Example 1 and confirmed to be pAMβ1 No. 1.

Example 13

As a culture medium, there was employed the same medium as described in Example 1 except that lactose was substituted for glucose.

0.2 ml of the culture solution obtained by a stationary culture of B. longum 108A strain at 37° C overnight was inoculated onto 10 ml of the above fresh medium and a stationary culture was effected at 37° C. When a turbidity of the medium reached $A_{600} = 0.2$, it was centrifuged at 3000 rpm at 4° C for 10 minutes to collect the bacteria. To the precipitate comprising the resultant bacteria was added an ice-cooled buffer [8 mM sodium phosphate buffer (pH 7.0) containing 0.4 M sucrose] and then the suspension was washed and centrifuged at 3000 rpm at 4° C for 10 minutes to collect the bacteria. The washing and collecting procedures were further repeated twice and the resulting bacteria were suspended in a buffer at a 1 100 volume of the culture solution to produce a bacteria suspension.

Separately, a mixture of a solution of the plasmid pAMβ1 No. 1 having erythromycin resistant gene with distilled water to make up a total of 50 μl was blended with 250 μl of a 40% aqueous solution of polyethylene glycol 6000. The resulting mixture was kept under ice-cooling.

To this mixture were added 100 μl of the bacteria suspension, the resulting mixture was mixed sufficiently, put into a cuvette equipped with a Gene Pulser and ice-cooled for 10 minutes. Then, an electric pulse of 5 kv/cm and 1 μF was applied. After application of the electric pulse, the mixture was again ice-cooled for 10 minutes, placed into a test tube and centrifuged at 15000 rpm at 4° C for 2 minutes to collect the bacteria. A suspension of the resulting precipitate in 1 ml of the medium was kept at 37° C for 3 hours, sprayed onto plates at the rate of 0.4 ml per plate and incubated anaerobically at 37° C.

A negative control was prepared by a similar electric pulse application without adding any plasmid and anaerobic incubation of plates spread with the suspension at the rate of 0.4 ml/plate at 37° C.

The numbers of erythromycin resistant colonies produced after 3 and 7 days are shown below in terms of the number of colonies per plate.

| Plasmid (μg) | None (0) | pAMβl No.1 (2) |
|---|---|---|
| After 3 days | 1 | 11 |
| After 7 days | 3 | 23 |

The above table shows that the bacteria subjected to electric pulse application in the coexistence of DNA could attain a remarkable erythromycin resistance.


Example 14


The same procedure as described in Example 13 was repeated except that polyethylene glycol 2000 was employed.

The numbers of erythromycin resistant colonies produced after 3 and 6 days are shown below.

| Plasmid (μg) | None (0) | pAMβl No.1 (2) |
|---|---|---|
| After 3 days | 0 | 6 |
| After 6 days | 1 | 13 |


Example 15


The same procedure as described in Example 13 was repeated except that an amount of the plasmid added, the electric pulse applied and the sort of polyethylene glycol were changed as indicated below. The number of the erythromycin resistant colonies produced after 6 days and the procedure conditions are summarized below.

EP 0 319 690 A1

| Plasmid (μg) | pAMβl No.1 | | | | | |
|---|---|---|---|---|---|---|
| | (0) | (2) | (4) | (2) | (2) | (2) |
| Voltage (kv)/cm | 5 | 5 | 5 | 5 | 2.5 | 5 |
| Electric capacity (μF) | 1 | 1 | 1 | 1 | 1 | 0.25 |
| Polyethylene glycol | 6000 | 6000 | 6000 | 2000 | 6000 | 6000 |
| After 6 days | 5 | 85 | 34 | 103 | 12 | 25 |

**Claims**

1. A method of introducing a foreign DNA into the cells of bacteria belonging to the genus Lactobacillus or Bifidobacterium, which comprises applying pulse currents to a suspension containing the cells of bacteria belonging to said genus and a foreign DNA.

2. A method of claim 1 wherein the DNA is selected from the group consisting of pAMβ1 (Nos. 1 and 2), pBL1003, pBL1027 and pC194.

3. A method of claim 1 wherein the bacteria belonging to the genus Lactobacillus is at the early logarithmic phase.

4. A method of claim 1 wherein the bacteria belonging to the genus Bifidobacterium is at the early logarithmic phase.

5. A method of claim 1 wherein the bacteria is selected from the group consisting of Lactobacillus casei, Lactobacillus acidophilus, Lactobacillus bulgaricus, Lactobacillus lactis, Lactobacillus helveticus, Lactobacillus salivarius, Lactobacillus plantarum, Lactobacillus fermentum, Lactobacillus cellobiosus, Lactobacillus delbrueckii, Lactobacillus buchneri and Lactobacillus brevis.

6. A method of claim 1 wherein the bacteria is selected from the group consisting of Bifidobacterium bifidum, Bifidobacterium infantis, Bifidobacterium breve, Bifidobacterium adolescentis, Bifidobacterium dentium, Bifidobacterium longum, Bifidobacterium pseudolongum, Bifidobacterium thermophilum, Bifidobacterium asteroides and Bifidobacterium indicum.

7. A method of claim 1 wherein the suspension further contains polyethylene glycol.

11

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | FEMS MICROBIOLOGY LETTERS, vol. 52, July 1988, pages 127-132, Amsterdam, NL; THEA AUKRUST and INGOLF F. NES: "Transformation of Lactobacillus plantarum with the plasmid pTV1 by electroporation" * whole document * | 1,5 | C 12 N 15/00 // (C 12 N 15/00 C 12 R 1:225) |
| X | J. GEN. APPL. MICROBIOL., vol. 33, no. 3, 1987, pages 287-294, Tokyo, JP; WILLIAM M. REED: "Protoplast fusion of lactobacillus acidophilus and streptococcus lactis via electric field or chemical induction" * whole document * | 1,5,7 | |
| X | FEMS MICROBIOLOGY LETTERS, vol. 44, no. 2, October 1987, pages 173-177, Amsterdam, NL; BRUCE M. CHASSY and JEANNETTE L. FLICKINGER: "Transformation of Lactobacillus casei by electroporation" * whole document * | 1,5 | |
| Y | CA-A-1 208 146 (T.K. WONG) * page 4, lines 29-30; page 5, lines 14-30; page 7, lines 17-21; claims 1-5,10-14 * | 1 | TECHNICAL FIELDS SEARCHED (Int. Cl.4) C 12 N 15/00 C 12 R 1/225 |
| A | | 2-6 | |
| Y | EP-A-0 133 046 (KABUSHIKI KAISHA YAKULT HONSHA) * whole document * | 1 | |
| A | --- -/- | 3,5,7 | |

The present search report has been drawn up for all claims

| Place of search | Date of completion of the search | Examiner |
|---|---|---|
| BERLIN | 23-02-1989 | JULIA P. |

European Patent
Office

EUROPEAN SEARCH REPORT

Application Number

EP  88 11 7717

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 554 827  (SHINRYO CORPORATION) <br> * page 5, lines 20-30; claims 1-3 * <br> --- | 1,3,5 | |
| A | ANGEW. CHEMIE, vol 93, April 1981, pages 332-351, Weinheim; U. ZIMMERMANN et al.: "Zellen mit manipulierten Funktionen: Neue Perspektiven für Zellbiologie, Medizin und Technik" <br> * abstract, page 333, claim 1, lines 52-55; page 348, claim 1, lines 1-7; claim 2, lines 29-41 * <br> --- | 1-6 | |
| A | BIOTECHNOLOGY, vol. 3, no. 12, December 1985, pages 1099-1103, New York, US; R.D. SHILLITO et al.: "High efficiency direct gene transfer to plants" <br> * abstract; page 1100, claim 1; figure 1; page 1102, claim 1, discussion * <br> ----- | 1,7 | |
| | | | TECHNICAL FIELDS SEARCHED (Int. Cl.4) |

The present search report has been drawn up for all claims

| Place of search | Date of completion of the search | Examiner |
|---|---|---|
| BERLIN | 23-02-1989 | JULIA P. |